# EUROPEAN PATENT SPECIFICATION

(11) **EP 2 164 333 B1**
(45) Date of publication and mention of the grant of the patent: **26.10.2016**
(21) Application number: 08754373.2
(22) Date of filing: 13.05.2008
(51) Int. Cl.: A23K 10/18, A23K 50/40, A61K 39/125, A61K 39/245, A61K 39/12, A61K 39/00

(54) **COMPOSITIONS AND METHODS USEFUL FOR MODULATING IMMUNITY, ENHANCING VACCINE EFFICACY, DECREASING MORBIDITY ASSOCIATED WITH CHRONIC FHV-1 INFECTIONS, AND PREVENTING OR TREATING CONJUNCTIVITIS**
ZUSAMMENSETZUNGEN UND VERFAHREN, DIE SICH FÜR DIE MODULIERUNG DER IMMUNITÄT, DER FÖRDERUNG DER IMPFSTOFFWIRKSAMKEIT, DER ERNIEDRIGUNG DER MIT CHRONISCHEN FHV-1-INFEKTIONEN EINHERGEHENDEN MORBIDITÄT UND DER VORBEUGUNG ODER BEHANDLUNG VON KONJUNKTIVITIS EIGNEN
COMPOSITIONS ET PROCÉDÉS UTILES POUR MODULER UNE IMMUNITÉ, AMÉLIORER L'EFFICACITÉ D'UN VACCIN, DIMINUER LA MORBIDITÉ ASSOCIÉE À DES INFECTIONS CHRONIQUES PAR LE FHV-1, ET PRÉVENIR OU TRAITER LA CONJONCTIVITE

(30) Priority: 24.05.2007 US 805813
(43) Date of publication of application: 24.03.2010
(73) Proprietor: Nestec S.A., 1800 Vevey (CH)
(72) Inventor: KNORR, Ruth, F-80800 Sailly le Sec (FR); CAVADINI, Christoph, CH-1804 Corsier-sur-Vevey (CH); BENYACOUB, Jalil, CH-1010 Lausanne (CH); SATYARAJ, Ebenezer, Wildwood, MO 63040 (US)
(74) Representative: Rupp, Christian
(86) International application number: PCT/US2008/006057
(87) International publication number: WO 2008/153655

(56) References cited:
- WO-A2-97/28812
- US-A1- 2005 106 133
- US-A1- 2007 098 744
- BENYACOUB JALIL ET AL: "Supplementation of food with Enterococcus faecium (SF68) stimulates immune functions in young dogs" JOURNAL OF NUTRITION, WISTAR INSTITUTE OF ANATOMY AND BIOLOGY, PHILADELPHIA, PA, US, vol. 133, no. 4, 1 April 2003 (2003-04-01) , pages 1158-1162, XP002415805 ISSN: 0022-3166
- BENYACOUB J ET AL: "Enterococcus faecium SF68 enhances the immune response to Giardia intestinalis in mice" JOURNAL OF NUTRITION, WISTAR INSTITUTE OF ANATOMY AND BIOLOGY, PHILADELPHIA, PA, US, vol. 135, no. 5, 1 May 2005 (2005-05-01), pages 1171-1176, XP002415804 ISSN: 0022-3166
- WEESE J SCOTT ET AL: "Bacteriological evaluation of dog and cat diets that claim to contain probiotics" CANADIAN VETERINARY JOURNAL - REVUE VETERINAIRE CANADIENNE, OTTAWA, CA, vol. 44, no. 3, 1 March 2003 (2003-03-01), pages 212-216, XP002415806 ISSN: 0008-5286
- ANDREW: 'Ocular manifestations of feline herpesvirus' JOURNAL OF FELINE MEDICINE & SURGERY vol. 3, no. 1, March 2001, pages 9 - 16, XP008129078

### SEQUENCE LISTING

<110> Knorr, Ruth Cavadini, Christoph Benyacoub, Jalil Satyaraj, Ebenezer
<120> Compositions comprising Probiotic
   Enterococci For Improved Immunity
<130> 48509-1015-00-US (NO 7991 US)
<140> 11/544,120
   <141> 2006-10-06
<150> US 60/724,214
   <151> 2005-10-06
<160> 1
<170> PatentIn version 3.3
<210> 1
   <211> 20
   <212> DNA
   <213> Artificial sequence
<220>
   <223> Primer
<400> 1
   ggttgggtga gaattgcacg

## Description

### BACKGROUND OF THE INVENTION

### Field of the Invention

The present invention relates generally to probiotic *Enterococcus bacteria E.faecium* strain NCIM 10415 (SF68) and particularly to the use of such probiotics to prevent or treat conjunctivitis.

### Description of the Related Art

Probiotics can be defined as live microorganisms that confer a health benefit to a host when administered in adequate amounts. It is theorized that probiotics may impart their beneficial health effects either by increasing the resistance to colonization of mucosal surfaces by pathogenic bacteria (colonization resistance) or by exerting a direct effect on gut associated lymphoid tissue (GALT) that promotes the production of immunomodulators.

Probiotics have been used to modulate the course of a variety of infectious diseases in human medicine. In contrast, few studies have been performed in veterinary medicine. The majority of veterinary studies have been in large animals where probiotics have been used to attempt to alter the shedding of fecal pathogens or to improve production parameters such as weight gain, feed conversion rate, and reduced mortality.

In one animal study, *Errterococcus faecium* strain NCIMB 10415 (SF68) was fed to a group of puppies vaccinated with canine distemper virus (CDV) and compared to a control group that received vaccinations only. Puppies supplemented with SF68 had increased serum and fecal total IgA concentrations, increased CDV-specifc IgG and IgA serum concentrations, and increased percentage of circulating B lymphocytes compared to control puppies proving an immune enhancing effect induced by this probiotic.

Feline herpesvirus 1 (FHV-1) infection is common in cats and extremely contagious between cats. FCV-1 frequently results in severe clinical disease in cats around the world. In the acute phase of infection, fever, sneezing, nasal discharge, conjunctivitis, cough, dyspnea, and death can occur. FHV-1 .persists as a latent infection after primary infection. While FHV-1 infected cats can be clinically normal for periods of time, the infection can be activated by crowding, other concurrent diseases, and other forms of stress. Recurrent conjunctivitis, keratitis, sneezing, and nasal discharge are common manifestations. In addition, during periods of activation, FHV-1 shedding rates are high, potentially resulting in the infection of other cats.

Feline panleukopenia (FPV) is a virus resulting in viremia followed by severe gastrointestinal disease. Appropriately vaccinated kittens have been shown to have sterilizing immunity. Feline rhinotracheitis (FHV-1) and feline calicivirus (FCV) are the two viral pathogens implicated in the syndrome. While FCV vaccines induce > 95% relative efficacy in vaccinates when compared to unvaccinated controls after being inoculated with a pathogenic challenge strain, FHV-1 vaccines only induce approximately 60% relative protection. Thus, FHV-1 continues to be a significant problem despite widespread vaccination. Previous attempts at improving vaccination efficacy have included intranasal administration. However, such administration leads to greater side effects and genetic manipulation of virulent strains which in turn leads to decreased disease severity but does not decrease the prevalence of the carrier state. The carrier state can lead to recrudescence or reinfection of the host as well as transmission to housemates. Multiple therapies for chronic FHV-1 infections have been tried, including interferon alpha, trephination, antiviral drugs, rhinotomy, glucocorticoids, topical decongestants, and antibiotics directed at secondary bacterial infections. However, administration of FHV-1 containing vaccines does not prevent infection and there are currently no drugs that eliminate FCV-1 from the body. The drugs used orally for treatment are expensive, can be ineffective, and can be toxic. None of these treatments has been able to clear the chronic viral infection. Therefore recurrences of viral shedding and clinical illness are common. Both cell-mediated and IgA mucosal immune responses are considered important in prevention and control of α-herpesvirus infections.

Conjunctivitis ("pinkeye" or Madras Eye") is an inflammation of the conjunctiva. Conjunctivitis is usually caused by allergic reactions or infections by bacterial or viral agents. Blepharoconjunctivitis is the combination of conjunctivitis with blepharitis and (inflammation of the eyelids). Keratoconjunctivitis is the combination of conjunctivitis and keratitis (corneal inflammation).

SE Andrew ("Ocular manifestations of feline herpesvirus" Journal of Medicine & Surgery, Vol. 3, No. 1, March 2001 (2001-03). pages 9-16) discloses that the virus FHV-1 is responsible for a number of different ocular manifestations including conjunctivitis, keratitis, stromal keratits, keratoconjunctivitis sicca, ophthalmia neonatorium, symblepharon, corneal sequestrum, eosinophilic keratitis and anterior uveitis, i.e. thereby assessing details of the overall nine different ocular manifestation that may be caused by feline herpesvirus-1 (FHV-1). SE Andrew lacks a disclosure of increasing the efficacy of therapy by administering probiotics of one or more Enterococcus bacteriae.

US 2007/0098744 A1 discloses compositions and methods for modulating the vaccine efficacy in animals against FHV-1 virus by utilizing probiotic organism, inter alia probiotic *Enterococcus* strains. US 2007/0098744 A1 is silent on a possible treatment of conjunctivitis but focuses generally on infections of cats with FHV-1 virus.

While there are several known methods for affecting immunity, vaccine efficacy, and FHV-1 infections, there still exists a need for new compositions and methods that improve innate and adaptive immunity, enhance vaccine efficacy, and decrease morbidity associated with chronic FHV-1 infections. Additionally, there is a need for compositions and methods for preventing or treating conjunctivitis in animals.

### SUMMARY OF THE INVENTION

It is, therefore, an object of the present invention to provide compositions that modulate immunity, enhance vaccine efficacy, decrease morbidity associated with chronic FHV-1 infections, and prevent or treat conjunctivitis.

This object is achieved by a a composition comprising Enterococcus bacteriae for use in preventing or treating conjunctivitis in an animal.

The probiotic in the present invention *Enterococcus spp.*

In is *Enterococcus faecium* NCIMB 10415 (SF68).

Other and further objects, features, and advantages of the present invention will be readily apparent to those skilled in the art.

### BRIEF DESCRIPTION OF THE DRAWINGS

Figure 1. Body weights (a) and fecal scores (b) over time of kittens supplemented with 150 mg chicken digest PO (Placebo, n=9) or 150 ing chicken digest mixed with 5 x 10⁸ cfu/day *Enterococcus faecium* strain NCIMB 10415 (SF68) (Treatment, n=9) daily starting at 7 weeks of age until 27 weeks of age. Kittens were vaccinated subcutaneously with a commercially available, modified live FHV-1 vaccine^{d} at 9 and 12 weeks of age. Box and whiskers represent the minimum, maximum, median and 25th and 75th percentiles. p>0.05 at all time points.
Figure.2. FHV-1 specific IgA results in serum (a) and saliva (b) from kittens with (Treatment) or without (Placebo) SF68 supplementation. Box and whiskers represent the minimum, maximum, median and 25th and 75th percentiles. p>0.05 for all time points.
Figure 3. FHV-1 specific IgG results in serum from kittens with (Treatment) or without (Placebo) SF68 supplementation. Box and whiskers represent the minimum, maximum, median and 25th and 75th percentiles. p>0.05 for all time points.
Figure 4. FCV specific IgG results from kittens with (Treatment) or without (Placebo) SF68 supplementation. Box and whiskers represent the minimum, maximum, median and 25th and 75th percentiles. p>0.05 for all time points.
Figure 5. FPV specific IgG results from kittens with (Treatment) or without (Placebo) SF68 supplementation. Box and whiskers represent the minimum, maximum, median and 25th and 75th percentiles. p>0.05 for all time points.
Figure 6. Total IgG (a) and IgA (b) in fecal extracts from kittens with (Treatment) or without (Placebo) SF68 supplementation. Box and whiskers represent the minimum, maximum, median and 25th and 75th percentiles. p>0.05 for all time points.
Figure 7. Percent of gated lymphocytes positive for CD4 (a) and CD8 (b) in peripheral blood by flow cytometry in kittens with (Treatment) or without (Placebo) SF68 supplementation. Box and whiskers represent the minimum, maximum, median and 25th and 75th percentiles. * denotes time points at which treatment group was significantly higher than placebo group.

### DETAILED DESCRIPTION OF THE INVENTION

### Definitions

The following abbreviations may be used in the specification and examples: FHV-1, feline rhinotracheitis virus; FCV, feline calcivirus; FPV, feline panleukopenia virus; spp., species; ELISA, enzyme linked immunosorbent assay; DM, dry matter; CFU, colony forming units; kg, kilogram; and BW, body weight

"Animal" means any animal that can benefit from an improved innate and adaptive immunity, enhanced vaccine efficacy, or decreased morbidity associated with chronic FHV-1 infections or any animal susceptible to or suffering from conjunctivitis. The methods and compositions of the invention are useful for a variety of human and non-human animals, including avian, bovine, canine, equine, feline, hicrine, murine, ovine, and porcine animals, and are particularly useful for companion animals such as canines and felines, including dogs and cats.

"Adaptive immunity" or "adaptive immune response" are used interchangeably and in a broad sense herein and mean the immune response to antigen challenge, including the development of immunological memory. The adaptive immune response includes, without limitation, humoral and cellular immunity.

"Cellular immunity" or "cellular immune response" or "cell mediated immunity" are used interchangeably herein and mean the activation of cytotoxic or helper T-lymphocytes, mononuclear cells, and cytokines in response to an antigen challenge. The term encompasses all adaptive immunity that cannot be transferred to a naïve recipient with antibodies.

"Companion animal" means any domesticated animal, and includes, without limitation, cats, dogs, rabbits, guinea pigs, ferrets, hamsters, mice, gerbils, horses, cows, goats, sheep, donkeys, pigs, and the like. Dogs and cats are most preferred, and cats are exemplified herein.

"Complete and nutritionally balanced pet or animal food" means a food that contains all known required nutrients in appropriate amounts and proportions based on recommendations of recognized authorities in the field of animal nutrition, and are therefore capable of serving as a sole source of dietary intake to maintain life or promote production, without the addition of supplemental nutritional sources. Nutritionally balanced pet food and animal food compositions are widely known and widely used in the art.

"Conjunctivitis medication" means any compound, composition, or drug useful for preventing or treating conjunctivitis, other than the composition of the present invention.

"Dietary supplement" means a product that is intended to be ingested in addition to the normal diet of an animal.

"Effective amount" means an amount of a compound, material, or composition effective to achieve a particular biological result. Such results include, but are not limited to, modulating immunity, enhancing vaccine efficacy, or decreasing morbidity associated with chronic FHV-1 infections in an animal. Such effective activity may be achieved, for example, by administering the compositions of the present invention to the animal.

"Food product formulated for human consumption" means any composition intended for ingestion by a human.

"Humoral immunity" or "humoral immune response" are used interchangeably herein and mean the production of immunoglobulin molecules in response to an antigen challenge.

"In conjunction" means that one or more probiotics or other compounds or compositions of the present invention are administered to an animal (1) together in a food composition or supplement or (2) separately at the same or different frequency using the same or different administration routes at about the same time or periodically. "Periodically" means that the medication is administered on a dosage schedule acceptable for a specific medication and that the probiotic is administered to an animal routinely as appropriate for the particular animal. "About the same time" generally means that the probiotic and medication are administered at the same time or within about 72 hours of each other. "In conjunction" specifically includes administration schemes wherein medication is administered for a prescribed period and the probiotics are administered indefinitely.

"Innate immunity" means the body's non-specific mechanisms for resistance to pathogens that are not enhanced upon subsequent challenge with a particular antigen.

"Modulate immunity" or "modulation of immunity" mean any enhancement or inhibition of the body's ability to generate an innate or adaptive immune response to antigen challenge, as measured by any means suitable in the art.

"Pet food" or "pet food composition" or "animal food" or "animal food composition" mean a composition that is intended for ingestion by an animal, particularly by companion animals.

"Probiotic(s)" means any organism, particularly microorganisms, that exerts a beneficial effect on the host animal such as increased health or resistance to disease. Probiotics can exhibit one or more of the following non-limiting characteristics: non-pathogenic or non-toxic to the host; are present as viable cells, preferably in large numbers; capable of survival, metabolism, and persistence in the gut environment (e.g., resistance to low pH and gastrointestinal acids and secretions); adherence to epithelial cells, particularly the epithelial cells of the gastrointestinal tract; microbicidal or microbistatic activity or effect toward pathogenic bacteria; anticarcinogenic activity; immune modulation activity, particularly immune enhancement; modulatory activity toward the endogenous flora; enhanced urogenital tract health; antiseptic activity in or around wounds and enhanced would healing; reduction in diarrhea; reduction in allergic reactions; reduction in neonatal necrotizing enterocolitis; reduction in inflammatory bowel disease; and reduction in intestinal permeability.

"Single package" means that the components of a kit are physically associated in or with one or more containers and considered a unit for manufacture, distribution, sale, or use. Containers include, but are not limited to, bags, boxes, bottles, shrink wrap packages, stapled or otherwise affixed components, or combinations thereof. A single package may be containers of individual probiotics, food compositions, and the like physically associated such that they are considered a unit for manufacture, distribution, sale, or use.

"Vaccine efficacy" means the ability of a vaccine to produce a desired therapeutic or protective effect on an animal against a specified pathogen. "Enhanced vaccine efficacy" refers to any improvement in the ability of a vaccine to produce a desired therapeutic or protective effect on an animal against a specified pathogen, as measured by any means suitable in the art.

"Virtual package" means that the components of a kit are associated by directions on one or more physical or virtual kit components instructing the user how to obtain the other components, e.g., in a bag containing one component and directions instructing the user to go to a website, contact a recorded message, view a visual message, or contact a caregiver or instructor to obtain instructions on how to use the kit.

All percentages expressed herein are by weight of the composition on dry matter basis unless specifically stated otherwise. The term "dry matter basis" means that an ingredient's concentration in a composition is measured after any moisture in the composition is removed.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "comprises", and "comprising" are to be interpreted inclusively rather than exclusively.

Unless defined otherwise, all technical and scientific terms and any acronyms used herein have the same meanings as commonly understood by one of ordinary skill in the art in the field of the invention: Although any compositions, methods, articles of manufacture, or other means or materials similar or equivalent to those described herein can be used in the practice of the present invention, the preferred compositions, methods, articles of manufacture, or other means or materials are described herein.

### The Invention

In one aspect, the present invention provides compositions suitable for modulating immunity, enhancing vaccine efficacy, decreasing morbidity associated with chronic FHV-1 infections, and/or preventing or treating conjunctivitis in animals. The compositions comprise as a probiotics *Enterococcus bacteria E. faecium* strain NCIMB 10415 (SF68) in an amount effective for preventing or treating conjunctivitis in the animals.

While not bound by theory, it is believed that administering probiotics such as *Enterococcus faecium* NCIMB 10415 (SF68) to an animal increases the number of CD4+ lymphocytes and affects immunity and the progression of certain infections and diseases.

Probiotics also mentioned herein comprise at least one of any suitable strain or subspecies of *Enterococcus. Enterococcus* species include, without limitation, *Enterococcus faecium, specifically E.faecium* strain NCIMB 10415 (SF68), as well as other Enterococci such as *E. faecium* DSM 10663 (M74), *E*. *faecium* GHR 017 DSM 7134, *E. faecium* CECT 4515, *E. faecium* CL15/ATCC 19434, *E. faecium* NCIMB 11181/DSM 5464, *E. faecium* IMB 52/DSM *3530, E. faecium* CNCM MA 17/5U*, E. faecium* 202 DSM 4788/ATCC53519, *E. faecium* 301 DSM 4789/ATCC 55593, *E. faecium* ATCC 19434, *E*. *faecium* EF-101 ATCC 19434, and *E faecium* AK 2205 BCCM/LMG S-16555.

In one embodiment, the compositions of the invention are pet or animal food compositions. These will advantageously include foods intended to supply necessary dietary requirements and foods such as treats and toys that supplement the diet. Optionally, the pet or animal food compositions can be a dry composition, semi-moist composition, wet composition, or any mixture thereof. In particular embodiments, the compositions are formulated for consumption by a feline, including but not limited to a domestic cat.

In another embodiment, the compositions of the invention are food products formulated for human consumption. These will advantageously include foods and nutrients intended to supply necessary dietary requirements of a human being as well as other human dietary supplements. In a detailed embodiment, the food products formulated for human consumption are complete and nutritionally balanced.

In another embodiment, the composition is a dietary supplement, such as gravy, drinking water, beverage, liquid concentrate, yogurt, powder, granule, paste, suspension, chew, morsel, treat, snack, pellet, pill, capsule, tablet, or any other delivery form. The dietary supplements can be specially formulated for consumption by a particular animal, such as companion or non-companion animal, particularly a feline, or a human. In one detailed embodiment, the dietary supplement can comprise a high concentration of probiotics such that the supplement can be administered to the animal in small amounts, or in the alternative, can be diluted before administration to an animal. The dietary supplement may require admixing with water prior to administration to the animal. IN one embodiment the supplement is in a sachet, alone or admixed with other probiotics or other materials.

The composition may be refrigerated or frozen. The probiotics may be pre-blended with the other components of the composition to provide the beneficial amounts needed, may be coated onto a pet food composition, dietary supplement, or food product formulated for human consumption, or may be added to the composition prior to offering it to the animal, for example, using a powder or a mix.

The compositions of the invention comprise probiotics in an amount effective for preventing or treating conjunctivitis. Pet foods and food products formulated for human consumption can be formulated to contain probiotics in the range of about 10² to about 10¹¹ colony forming units (CFU) per gram of composition. Dietary supplements may be formulated to contain several fold higher concentrations of probiotic, to be amenable for administration to an animal in the form of a tablet, capsule, liquid concentrate, or other similar dosage form, or to be diluted before administrations, such as by dilution in water, spraying or sprinkling onto a pet food, and other similar modes of administration.

In one embodiment, the concentration of probiotics in the composition is a function of the amount required to modulate immune functions, including an increase in the proportion and/or numbers of CD4+ lymphocytes in the blood of the animal. In another embodiment, the concentration of probiotics in the composition is a function of an amount required to increase the concentration of immunoglobulins reactive against antigens of a specified pathogen in the blood serum, feces, and secretions such as milk, tears, and saliva. The level of CD4+ lymphocytes and the concentration of immunoglobulins in the blood serum, feces, and secretions such as milk, tears, and saliva of the animal may be determined by any means recognized and appreciated by one of skill in the art.

The compositions of the invention can optionally comprise supplementary substances such as minerals, vitamins, salts, condiments, colorants, and preservatives. Non-limiting examples of supplementary minerals include calcium, phosphorous, potassium, sodium, iron, chloride, boron, copper, zinc, magnesium, manganese, iodine, selenium and the like. Non-limiting examples of supplementary vitamins include vitamin A, various B vitamins, vitamin C, vitamin D, vitamin E, and vitamin K. Additional dietary supplements may also be included, for example, niacin, pantothenic acid, inulin, folic acid, biotin, amino acids, and the like.

The compositions of the invention can optionally comprise one or more supplementary substances that promote or sustain a healthy immune system, or further modulate immunity. In one embodiment, the compositions of the invention further comprising one or more substances effective for modulating immunity, enhancing vaccine efficacy, or decreasing morbidity associated with chronic FHV-1 infections in animals. Such substances include, without limitation, L-arginine, steroids such as 7-oxo Dehydroepiandrosterone (7-oxy DHEA), carotenoids such as alpha- and beta-carotene, antioxidants, and herbs or herbal extracts such as astragalus and echinacea.

In various embodiments, animal food or dietary supplement compositions of the invention can comprise, on a dry matter basis, from about 15% to about 50% crude protein, by weight of the composition. The crude protein material may comprise vegetable proteins such as soybean, cottonseed, and peanut, or animal proteins such as casein, albumin, and meat protein. Non-limiting examples of meat protein useful herein include pork, lamb, equine, poultry, fish, and mixtures thereof.

The compositions may further comprise, on a dry matter basis, from about 5% to about 40% fat, by weight of the composition. The compositions may further comprise a source of carbohydrate. The compositions may comprise, on a dry matter basis, from about 15% to about 60% carbohydrate, by weight of the composition. Non-limiting examples of such carbohydrates include grains or cereals such as rice, corn, sorghum, alfalfa, barley, soybeans, canola, oats, wheat, and mixtures thereof. The compositions may also optionally comprise other materials such as dried whey and other dairy by-products.

The compositions may also comprise at least one fiber source not exceeding 1% of the final food composition. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, pectin, fructooligosaccharide additional to the short chain oligofructose, mannanoligofructose, soy fiber, fiber from lupins, arabinogalactan, galactooligosaccharide, arabinoxylan, or mixtures thereof. Alternatively, the fiber source can be a fermentable fiber. Fermentable fiber has previously been described to provide a benefit to the immune system of companion animals. Fermentable fiber or other compositions known to those of skill in the art which provide a prebiotic composition that could enhance the growth of probiotic microorganisms within the intestine may also be incorporated into the composition to aid in the enhancement of the benefit provided by the present invention to the immune system of an animal.

In one embodiment, the composition is a complete and nutritionally balanced pet or animal food. In this context, the pet food may be a wet food, a dry food, or a food of intermediate moisture content, as would be recognized by those skilled in the art of pet food formulation and manufacturing. "Wet food" describes pet food that is typically sold in cans or foil bags, and has a moisture content typically in the range of about 70% to about 90%. "Dry food" describes pet food which is of a similar composition to wet food, but contains a limited moisture content, typically in the range of about 5% to about 15%, and therefore is presented, for example, as small biscuit-like kibbles. The compositions and dietary supplements may be specially formulated for adult animals, or for older or young animals, for example, a "puppy chow," "kitten chow," or "senior" formulation. In general, specialized formulations will comprise energy and nutritional requirements appropriate for animals at different stages of development or age.

Certain aspects of the invention are preferably used in combination with a complete and balanced food (for example, as described in National Research Council, 2006, Nutritional Requirements for Dogs and Cats, National Academy Press, Washington D.C., or Association of American Feed Control Officials, Official Publication 1996). That is, compositions comprising probiotics according to certain aspects of this invention are preferably used with a high-quality commercial food. As used herein, "high-quality commercial food" refers to a diet manufactured to produce the digestibility of the key nutrients of 80% or more, as set forth in, for example, the recommendations of the National Research Council above for dogs and cats, or in the guidelines set forth by the Association of American Feed Control Officials. Similar high nutrient standards would be used for other animals.

The skilled artisan will understand how to determine the appropriate amount of probiotics to be added to a given composition. Such factors that may be taken into account include the type of composition (*e.g*., pet food composition, dietary supplement, or food product formulated for human consumption), the average consumption of specific types of compositions by different animals, and the manufacturing conditions under which the composition is prepared. The concentrations of probiotics to be added to the composition can be calculated on the basis of the energy and nutrient requirements of the animal. According to certain aspects of the invention, the probiotics can be added at any time during the manufacture and/or processing of the composition. This includes, without limitation, as part of the formulation of the pet food composition, dietary supplement, or food product formulated for human consumption, or as a coating applied to the pet food composition, dietary supplement, or food product formulated for human consumption.

The compositions can be made according to any method suitable in the art such as, for example, that described in Waltham Book of Dog and Cat Nutrition, Ed. ATB Edney, Chapter by A. Rainbird, entitled "A Balanced Diet" in pages 57 to 74, Pergamon Press Oxford

In various embodiments of the composition, the probiotics are in the composition as an ingredient or as an additive or are on the composition, e.g., sprayed on the composition.

The probiotics are administered using any method for administering probiotics to an animal. In one embodiment, the probiotics are administered orally using a composition of the present invention, including a pet or animal food composition, dietary supplement, or food product formulated for human consumption.

Wherein a human is directed to feed the composition to an animal, such direction may be that which instructs and/or informs the human that use of the composition may and/or will provide the referenced benefit, for example, the modulation of immunity or enhancement of vaccine efficacy in the animal. Such direction may be oral direction (e.g., through oral instruction from, for example, a physician, veterinarian, or other health professional, or radio or television media (*i.e*., advertisement), or written direction (*e.g*., through written direction from, for example, a physician, veterinarian, or other health professional *(e.g.,* prescriptions), sales professional or organization (*e.g.,* through, for example, marketing brochures, pamphlets, or other instructive paraphernalia), written media (e.g., internet, electronic mail, or other computer-related media), and/or packaging associated with the composition (*e.g.,* a label present on a container holding the composition).

Administration can be on an as-needed or as-desired basis, for example, once-monthly, once-weekly, daily, or more than once daily. Similarly, administration can be every other day, week, or month, every third day, week, or month, every fourth day, week, or month, and the like. Administration can be multiple times per day. When utilized as a supplement to ordinary dietetic requirements, the composition may be administered directly to the animal or otherwise contacted with or admixed with daily feed or food. When utilized as a daily feed or food, administration will be well known to those of ordinary skill.

Administration can also be carried out on a regular basis, for example, as part of a diet regimen in the animal. A diet regimen may comprise causing the regular ingestion by the animal of a composition comprising one or more probiotics in an amount effective to modulate immunity or to enhance vaccine efficacy in the animal. Regular ingestion can be once a day, or two, three, four, or more times per day, on a daily or weekly basis. Similarly, regular administration can be every other day or week, every third day or week, every fourth day or week, every fifth day or week, or every sixth day or week, and in such a dietary regimen, administration can be multiple times per day. The goal of regular administration is to provide the animal with the preferred daily dose probiotics, as exemplified herein.

As also described herein, administration of the compositions comprising one or more probiotics, including administration as part of a diet regimen, can span a period of time ranging from gestation through the entire life of the animal.

In one embodiment, the probiotics modulate innate immunity in the animal. In another, the probiotic modulate the adaptive immune response in the animal. In a further, the probiotics enhance the efficacy of vaccines administered to an animal. In another, the probiotics decrease morbidity associated with chronic FHV-1 infections. In a further, the probiotics prevent or treat conjunctivitis. In a preferred embodiment, the probiotics enhance the efficacy of vaccines against FHV-1, FCV, and FPV in the animal.

In some embodiments, the vaccine is for feline panleukopenia virus, feline rhinotracheitis virus, or feline calcivirus.

The daily dose of probiotics can be measured in terms of colony forming units (CFU) administered per animal, per day. The daily dose of probiotics can range from about 10⁵ to about 10¹² CFU/day. More preferably, the daily dose of probiotics is about 10⁷ to about 10⁹ CFU/day. More preferably, the daily dose of probiotics is about 10⁸ to about 10⁹ CFU/day. Most preferably, the daily dose of probiotics is about 10⁸ CFU/day. In one embodiment, the probiotics are administered in conjunction with one or more conjunctivitis medication.

In particular, the present invention relate to members of the Felidae, the cat family, to which the invention may be applied in instances where the cat is available to receive administration of the probiotic composition (*e.g*., in a zoo, veterinary facility, game preserve, and the like). In addition to the domestic cat, *Felis cattus,* the Felidae include members of the genera: (1)*Acinonyx,* such as the cheetah (*A. fubatus),* (2) *Neofelis,* such as the clouded leopard *(N. nebulosa),* (3) *Panthera,* such as the lion (*P. leo),* jaguar (*P. onca*)*,* leopard (P. pardus), tiger (P. tigris); (3) *Uncia,* such as the snow leopard (*U. uncial*); (4) *Puma,* such as the cougar, mountain lion or puma (*P. concolor*) and (5) various species of non-domesticated cats (*Felis*)*,* including but not limited to Bornean bay cat (*F. badia),* Caracal *(F. caracal),* Chinese mountain cat (*F. bieti*), jungle cat (*F. chaus*)*,* sand cat (*F. margarita*)*,* black-footed cat (*F. nigripes*), wildcats (*F. sylvestris, F. lybica),* jaguarondi *(F. yagouraroundi*), ocelot (*F. pardalis),* oncilla (*F. tigrina*), margay (*F. wieldi*), serval (*F*. *serval),* lynx (*F. lynx*)*,* bobcat (*F. rufus*)*,* pampas cat (*F. colocolo*)*,* Geoffroy's cat (*F. geoffroyi*)*,* Andean mountain cat (*F. jacobita*)*,* pallas cat (*F. manul*), kodkod (*F. guigna*), leopard cat (*F. bengalensis*, *F*. *iriomotensis),* flat-headed cat (*F. planiceps*), rusty-spotted cat (*F. rubiginosus*), fishing cat (*F. viverrina*)*,* and African golden cat *(F. aurata).* As used herein, the term "feline" refers to members of the cat family unless specified otherwise.

In a further aspect, the present invention allows for a use of a composition comprising probiotic *Enterococcus* bacteria *E.faecium* strain NCIMB 10415 (SF68) to prepare a medicament. In another, the invention allows for the use of such composition to prepare a medicament for preventing or treating conjunctivitis in animals. Generally, medicaments are prepared by admixing one or more probiotics of the invention with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal. The medicament contains the probiotics in amounts useful for preventing or treating conjunctivitis in animals.

### EXAMPLES

The invention can be further illustrated by the following examples, although it will be understood that these examples are included merely for purposes of illustration and are not intended to limit the scope of the invention unless otherwise specifically indicated

### Example 1

### Animals and Experimental Parameters

Feline study population. Twenty, six-week old SPF kittens were purchased from a Liberty Laboratories (Liberty, NY). The kittens were shown to be seronegative for feline leukemia virus antigen and feline immunodeficiency virus antibodies by ELISA. (Snap Combo, IDEXX Laboratories, Portland, ME).

Experimental design. After a 10 day equilibration period, the kittens were randomized into two groups of ten kittens each and the treatment study started at 7 weeks of age. Between 0.25 and 0.28 g (∼ 5 x 10⁹ CFU based on dilution count assays) of LBC ME5 PET *E. faecium* NCIMB 10415 (SF68) (Cerbios-Pharma SA, Switzerland) were added into individual 50 mL conical bottom polypropylene centrifuge tubes, capped, and stored at 4°C for the duration of the study. Similar preparations were used for aliquots of the palatability enhancer (a typical pet food coating comprising liver digest as the main component was used) using 150 mg per tube. Aliquots were monitored for water absorption and were to be discarded if there appeared to be any clumping of either the probiotic or palatability enhancer, Just before administration, one aliquot of palatability enhancer was transferred to one of the stored SF68 tubes (treatment group) or an empty tube (placebo group) and diluted using room temperature tap water to a total volume of 10 mL. Contents were vortexed for at least three minutes and aspirated into a 12 cc syringe. Immediately after vortexing the suspension, appropriate kittens were orally administered 1 ml of either the SF68 (total daily dose 5x10⁸ CFU per day) or the palatability enhancer alone (placebo kittens) until they were 27 weeks of age. Both groups were fed dry kitten food *ad libitum* (typical kitten growth formula meeting all AAFCO requirements and was based on chicken and rice as main ingredients was used) and gang housed in two separate rooms to avoid cross-contamination with the probiotic. At 9 and 12 weeks of age, all kittens were vaccinated subcutaneously with a modified live combination vaccine (Pfizer Animal Health, Exton, PA) for feline herpesvirus-1, calicivirus, and panleukopenia virus as recommended by the American Association of Feline Practitioners. (Richards J *et al*. (2001)).

Statistical evaluation. On each sample date, group mean values for all measured parameters were calculated. Differences between the probiotic-treated group and placebo group were analyzed using a mixed ANOVA model appropriate for a repeated measures experiment. Time was included in the model as a continuous variable. Percentages of cat samples positive for *C.perfringens* enterotoxin or C. *difftcile* toxins A or B and percentages of gated cells positive for cell surface markers were calculated for each group of cats over the duration of the study and compared by a two tailed t test (GRAPHPAD Prism, GRAPHPAD Software, Inc., San Diego, CA). Statistical significance was considered to be p < 0.05.

### Example 2

### Sample Collection and Clinical Monitoring

The attitudes and behavior of the kittens were monitored daily throughout the study. Body weight was measured weekly. Blood, saliva, and feces were collected from all cats prior to starting probiotic or palatability enhancer supplementation at 7 weeks of age and at 9, 15, 21, and 27 weeks of age. In addition, feces were collected from kittens in the treatment group at 28 weeks of age. For each group of kittens, 5 fecal samples per day were randomly selected from the shared litterbox and scored using a standardized graphic scoring card and the daily group means determined. Fecal extracts for total IgA and total IgG measurement were processed according to the protocol described by Benyacoub J *et al.* (2003)). All samples were stored at-80°C until assayed in batches.

The stools of all kittens were normal at the beginning of the supplementation period (7 weeks of age). One kitten in each group was removed from the study for reasons unrelated to the study and were therefore removed from the final data analysis. Body weight and fecal scores were not statistically different between the two groups over time or at any individual time points (Figure 1).

Complete blood cell counts, biochemistry parameters, and body weights were similar between groups of cats over the course of the study. Fecal scores were similar between groups as well suggesting that use of SF68 at the dosage described here will induce no noticeable clinical abnormalities.

### Example 3

### Fecal Assays

On each sample date, feces from each kitten were plated in eight serial 10-fold dilutions onto KF Streptococcus Agar and incubated for 48 hours at 37°C aerobically. Ten colonies of each morphology type were picked off using sterile loops and placed in 1.2 mL brain heart infusion medium (BHI) (Becton Dickinson, Franklin Lakes, NJ) and stored at -80°C pending analysis. RAPD-PCR was performed on bacterial isolates from each sample to determine if viable *E. faecium* NCIMB 10415 (SF68) was in the stools of treated cats and to assess whether the probiotic was accidentally transmitted from the treated kittens to the control kittens. The thermocycler parameters were as follows: 30 cycles of one minute of denaturation at 95°C, one minute of annealing 40°C, four minutes extension at 72°C. The 25.5 µL reaction mixture included 2.45 µL 10x *magnesium-free buffer (100 mM Tris-HCl, pH 8.3, 500 mM KCl), 3.22 mM MgCl2, 0.4 µL (1 Unit), JumpStart Taq DNA polymerase (Sigma D-4184, Sigma-Aldrich, Inc., St. Louis, MO), 1.9 µL dNTP mix (2.5- mM), 1 µL primer (100 µM), 15.47 µL PCR water, and 1 µL bacterial culture. The sequence of the primer used was 5'-GGTTGGGTGAGAATTGCACG-3'. Five to ten µL of the PCR product was run on a two percent agarose gel and patterns of banding were compared to a positive SF68 control. Commercially available ELISAs were used to determine whether Clostridium perfringens enterotoxins or C. difficile toxins A/B were present in the feces of all kittens. (*C. perfiringens* (ELISA, Kit No. 92-000-22) and C. *difficile* (ELISA, KitNo. 94-0150-KT), Techlabs, Blacksburg, VA.) Routine aerobic fecal cultures for *Salmonella* spp. and *Campylobacter* spp. were performed by the Colorado State University Diagnostic Laboratory.

Feces from seven of nine treatment cats were positive for SF68 on at least one time point during the study. However, SF68 DNA was not amplified from feces of any treated cat 1 week after stopping supplementation (week 28). Neither *Salmonella* spp. nor *Campylobacter* spp. were grown from feces. All samples from placebo cats were negative for SF68 by RAPD PCR. Numbers of positive samples for *C. difficile* toxins A/B or *C. perfringens* enterotoxin (Table 1) did not vary between the groups over the course of the study.

*Salmonella spp.* and *Campylobacter spp,* shedding was not induced by SF68 supplementation. Several fecal samples in both groups of kittens were positive for *C*. *difficile* or C. *perfringens* toxins; however, there was no significant difference in number of positive samples between groups and positive results did not correlate to the presence of diarrhea. SF68 was detected in the feces of the majority of treated cats during the period of supplementation, but was no longer detected in the feces 1 week after stopping supplementation indicating that the organism persisted in the cats only transiently. Thus, administration of SF68 using the dosage described herein has no deleterious effects and is safe for administration in the time period studied.

### Example 4

### Immunologic Assays

Complete blood counts, serum biochemical panels, and urinalyses were performed at the Clinical Pathology Laboratory at Colorado State University. Antigen specific humoral immune responses were estimated by measuring serum FHV-1-specific IgG, FHV-1-specific IgA, FCV-specific IgG, and feline panleukopenia-specific IgG in sera as well as FHV-1 specific IgG and IgA levels in saliva using adaptations of previously published ELISA assays. (Lappin *MR et al.* (2002); and Ditmer DA *et al.* (1998). For FHV-1 specific IgG and IgG, results were calculated by both the mean absorbance for the triplicate test wells for each sample and by calculation of percentage ELISA units (test sample mean absorbance minus the negative control sample mean absorbance/positive control sample mean absorbance minus the negative control sample mean absorbance multiplied by 100). For FCV and FPV, mean absorbances were used. Total IgG and IgA concentrations in sera, fecal extracts, and saliva were estimated by use of commercially available ELISA assays or radial immunodiffusion assay. (Bethyl Laboaratories, Inc., Montgomery, TX).

Cellular immune responses were assessed via flow cytometry and whole blood proliferation assays. Flow cytometry was performed within 12 hours of blood collection using 500 µL of anticoagulated (EDTA) blood incubated at room temperature in red cell lysis buffer (0.155 M MH₄Cl/0.010 MKHCO₃/5 X 10⁻⁴% Phenol Red (0.5%). Cells were washed two times with PBS and the resultant cell pellets were resuspended in FACS buffer containing PBS, 0.1% sodium azide and 2% fetal bovine serum to attain a concentration of 1x10⁶ cells/100 µL if possible. Samples with insufficient cells for at least 500 µL of the above suspension were counted and cell concentration recorded. One hundred µL of each cell suspension was added to individual wells in a round-bottom 96 well plate for immunostaining. Non-specific binding was blocked by addition of 10% normal cat serum. (Jackson ImmunoResearch Laboratories, Inc., West Grove, PA). Immunostaining was done at 4°C in the dark in FACS buffer. Lymphocytes were stained for expression of CD4 and CD8 (vpg34; anti-CD4-FITC, vpg9; anti-CD8-RPE antibodies; Serotec, Raleigh, NC (Oxford, UK)) and expression of CD44 (IM7; anti-CD44-PE/CY5 antibody; Pharmingen, Franklin Lakes, NJ). For analysis of B cells, lysed whole blood was immunostained with cross-reactive antibodies to B220 (ra3-b62; anti-H220-biotinylated antibody; eBioscience, San Diego, CA), CD21 (b-ly4; anti-CD21-APC antibody; BD-Biosciences, Franklin Lakes, NJ), and MHC class II (anti-MHC class II-FITC antibody; clone CAG5-3D1, Serotec, Raleigh, NC (Oxford, UK)). Cells for analysis were gated on live lymphocyte populations based on forward and side-scatter characteristics. Data were collected on a Cyan MLE cytometerp and analyzed using Summit software. (Dako-Cytomation, Fort Collins, CO).

Proliferation assays were performed in triplicate using 10 µL whole heparinized blood preconditioned by incubating in 100 µL complete tumor media at 37°C with 5% CO2 for 30 minutes before addition of the mitogen or antigen. (Complete tumor media: modified Eagle's medium supplemented with essential and non-essential amino acids + 10% FBS). Cells were maintained in medium alone (unstimulated), or stimulated with concanavalin A (10 µglmL: Con A, Sigma-Aldrich, St Louis, MO), or a FHV-1 antigen preparation (1µL/well, prepared prior to the start of the study and stored aliquotted at -80°C) for 96 hours at 37°C with 5% CO2. (Veir JK *et al.* (2005)). Cells were pulsed with 1 µCi tritiated thymidine per well and harvested 18 hours later onto fiberglass filter mats. (Wallac-Microbeta Perkin -Elmer, Boston, MA). Mats were read using a MicroBetas liquid scintillation counter. The mean stimulation index (mean maximum count per stimulated sample divided by mean maximum count per unstimulated sample) was calculated for all samples.

Complete blood counts and biochemical profiles were within normal limits for the age group for all cats at all time points. There was no statistical difference between the groups over time or at any individual time points among the assays analyzed. At 21 and 27 weeks of age, the mean levels of FHV-1-specific IgA in serum and saliva were numerically greater in the treatment group when compared to the placebo group, but the differences were not statistically different (Figure 2). At 15, 21, and 27 weeks of age the mean FHV-1-specific serum IgG levels were numerically greater in the treatment group when compared to the placebo group using both assays, but the differences were not statistically significantly different (Figure 3). No FHV-1 specific IgG was detected in saliva. FCV-specific-IgG levels in serum were similar between groups (Figure 4). At 15 weeks of age, the treatment group serum mean FPV-specific IgG levels were numerically greater than those of the placebo group, but the differences were not statistically significantly different (Figure 5).

Concentrations of total IgG and IgA in serum were similar between groups (data not shown). Total IgG was not detected in saliva and total IgA concentrations in saliva were similar between groups (data not shown). At 27 weeks of age, the treatment group mean concentrations of total IgG in fecal extracts were numerically greater than those of the placebo group, but the differences were not statistically different (Figure 6). Total IgA concentrations in fecal extracts were similar between groups (Figure 6).

Proliferation assays using either 10 µg/mL concanavalin A or 1 µL FHV-1 antigen preparation as the stimulants did not produce significantly different mean maximum counts between groups at any time points. There were no statistical differences between the groups for any cell surface markers at the first four time points (Figure 7). At 27 weeks of age, the treatment group (mean 13.87%) had a significantly higher percentage of gated lymphocytes positive for CD4 than the placebo group (mean 10.61%, p = 0.0220). No other comparisons were significantly different.

The increase in CD4+ counts in the treatment group compared to the placebo group without a concurrent increase in CD8+ counts at 27 weeks of age demonstrates systemic immune modulating effects by the probiotic.

After vaccinations, each of the kittens developed FHV-1, FCV, and FPV-specific serum antibody responses that are similar to other studies indicating they were immunocompetent and that the modified live vaccine used was viable. (Lappin MR *et al.* (2002)). Several of the results also indicate that feeding of the probiotic influenced humoral and cell-mediated immune responses of these kittens. These include the detection of statistically significantly greater CD4+ lymphocytes counts at 27 weeks of age and numerically greater mean values for FHV-1-specific IgA in serum and saliva at 21 and 27 weeks of age, FHV-1-specific IgG levels in serum at 15, 21, and 27 weeks of age, FPV-specific IgG levels in serum at 15 weeks of age when compared to the placebo group.

### Example 5

### Effect of Probiotic Enterococcus Bacteria on Conjunctivitis

Young adult, mixed sex cats with chronic FHV-1 infection (n = 12) were transferred from another study and administered either SF68 or a placebo for a period of 5.5 months. The cats were monitored daily for conjunctivitis, sneezing, and nasal discharge during an equilibration period and after supplementation. During the supplementation period, the cats were moved from group housing to individual housing and surgically sterilized in an attempt to activate latent FHV-1 infection. The animals were monitored for conjunctivitis.

The results show that, overall, conjunctivitis was common, occurring in 122 of 750 (16.3%) and 210 of 755 (27.8%) of the observation points for SF68 treated cats and placebo cats, respectively. During the equilibration period, numbers of observation points where conjunctivitis was noted did not vary between the two groups but after initiating the administration of SF68 or placebo, SF68 supplemented cats had significantly less episodes of conjunctivitis than placebo cats within each treatment period. The results show that SF68 decreased the morbidity associated with chronic FHV-1 infections.

In the specification, there have been disclosed typical preferred embodiments of the invention and, although specific terms are employed, they are used in a generic and descriptive sense only and not for purposes of limitation, the scope of the invention being set forth in the claims.

## Claims

1. A composition comprising the probiotic *Enterococcus bacteria E. faecium* strain NCIMB 10415 (SF68) for use in preventing or treating conjunctivitis caused by FHV-1 in a feline.

2. The composition for use according to claim 1 wherein the composition is an animal food composition or dietary supplement.

3. The composition for use according to any preceding claim, wherein the probiotic *Enterococcus bacteria* is present in an amount of at least about 10² to about 10¹¹ colony forming units (CFU) per gram of the composition.

## Patentansprüche

1. Zusammensetzung, umfassend den probiotischen *Enterococcus bacteria E. faecium*-Stamm NCIMB 10415 (SF68) zur Verwendung beim Verhindern oder Behandeln von durch FHV-1 verursachter Konjunktivitis bei einer Katze.

2. Zusammensetzung zur Verwendung nach Anspruch 1, wobei die Zusammensetzung eine Tierfutterzusammensetzung oder -nahrungsergänzung ist.

3. Zusammensetzung zur Verwendung nach einem der vorstehenden Ansprüche, wobei die probiotische *Enterococcus bacteria* in einer Menge von wenigstens etwa 10² bis etwa 10¹¹ koloniebildenden Einheiten (KBE) pro Gramm der Zusammensetzung vorliegt.

## Revendications

1. Composition comprenant la souche NCIMB 10415 (SF68) du probiotique *Enterococcus bacteria E. faecium* pour une utilisation dans le cadre de la prévention ou du traitement de conjonctivite causée par FHV-1 chez un félin.

2. Composition pour utilisation selon la revendication 1, dans laquelle la composition est une composition alimentaire ou un complément diététique pour animaux.

3. Composition pour utilisation selon l'une quelconque des revendications précédentes, dans laquelle le probiotique *Enterococcus bacteria* est présent dans une quantité d'au moins environ 10² à environ 10¹¹ unités formant des colonies (UFC) par gramme de composition.
